(19) Europäisches Patentamt
European Patent Office
Office européen des brevets

(11) Veröffentlichungsnummer: **0 054 514**
**A1**

(12) # EUROPÄISCHE PATENTANMELDUNG

(21) Anmeldenummer: **81810489.5**

(22) Anmeldetag: **11.12.81**

(51) Int. Cl.³: **C 07 H 19/00**
A 61 K 31/70

(30) Priorität: **16.12.80 CH 9266/80**

(43) Veröffentlichungstag der Anmeldung:
**23.06.82 Patentblatt 82/25**

(84) Benannte Vertragsstaaten:
**BE CH DE FR GB IT LI LU NL SE**

(71) Anmelder: **CIBA-GEIGY AG**
**Patentabteilung Postfach**
**CH-4002 Basel(CH)**

(72) Erfinder: **Traxler, Peter, Dr.**
**Bündtenring 3**
**CH-4124 Schönenbuch(CH)**

(54) **Neue antibiotisch wirksame Aminopapulacandin-Derivate.**

(57) N-Acylierte 11-Aminoderivate des Papulacandins A oder B der Formel I

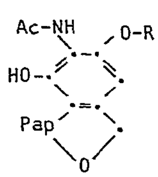

(1),

worin Pap den restlichen Teil des Papulacandins A oder B, R Methyl oder Wasserstoff und Ac den Acylrest einer organischen Säure bedeutet,

(z.B.
Mesyl oder ein Acylrest der Formel

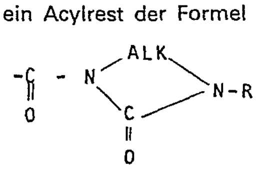

11 )

und die nicht acylierte 11-Aminoderivate, einschliesslich Salze von Verbindungen mit salzbildenden Eigenschaften, zeichnen sich durch ihre antibiotische, insbesondere antimykotische Wirkung aus und sind zur Bekämpfung von derartigen Infektionen, vor allem den durch Candida albicans verursachten, anwendbar. Die Verbindungen werden nach konventionellen Verfahren aus entsprechenden 11-unsubstituierten Papulacandin-Derivate hergestellt.

EP 0 054 514 A1

Croydon Printing Company Ltd.

CIBA-GEIGY AG
Basel (Schweiz)

4-13193/=

## Neue antibiotisch wirksame Aminopapulacandin-Derivate

Die vorliegende Erfindung betrifft antibiotisch wirksame Aminopapulacandin-Derivate, und insbesondere N-acylierte 11-Aminoderivate des Papulacandins A und B, die infolge ihrer guten antibiotischen Wirkung gegen Pilze, sowie auch als Zwischenprodukte zur Herstellung anderer antibiotisch wirksamer Papulacandin-Derivate, von Interesse sind.

In der deutschen Offenlegungsschrift 2'609'611 wurde ein neues Antibiotikum "A 32283" beschrieben, welches durch Züchtung des Stammes Papularia sphaerosperma (Pers.) Höhnel NRRL 8086 erhalten wird. Wie in jener Offenlegungsschrift offenbart wurde, besteht dieses Antibiotikum, das heute als "Papulacandin" bezeichnet wird, hauptsächlich aus zwei antibiotisch wirksamen Komponenten, nämlich zu etwa 70% aus einer Komponente B (Papulacandin B) und zu etwa 20% aus einer Komponente A (Papulacandin A), während der Rest (ca. 10%) mehrere strukturell verwandte Nebenkomponenten enthält.

Die endgültige Struktur von Papulacandin A und B wurde kürzlich ermittelt, vgl. P. Traxler, H. Fritz, H. Fuhrer und W. Richter: Journal of Antibiotics, 33(9), 967-978 (1980, und ist durch die nachstehende Formel P dargestellt:

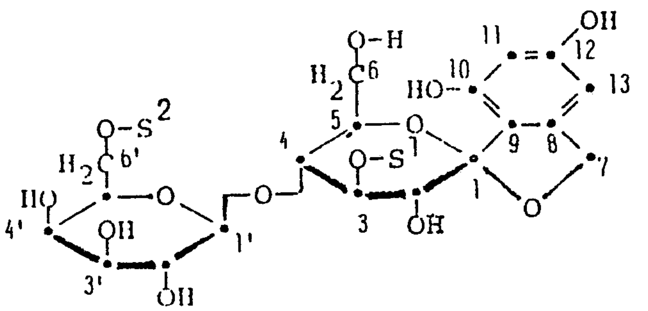

(P)

$S^1$ =

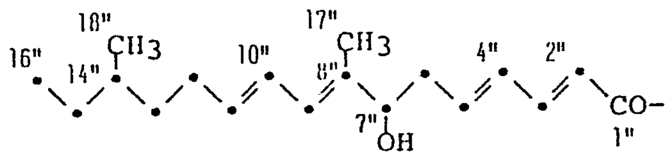

$S^2$ =

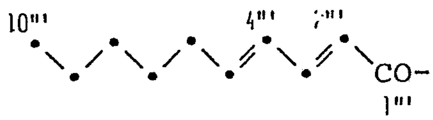

Papulacandin A - $S^2_A$

oder

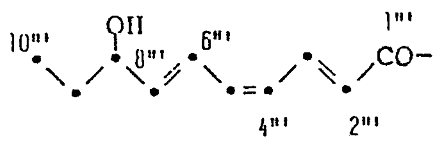

Papulacandin B - $S^2_B$

In der oben genannten Offenlegungsschrift wurden auch einige funktionelle Derivate des Papulacandins A und B beschrieben, unter anderem auch Ether, in denen eine oder beide der phenolischen Hydroxylgruppen mit Niederalkanolen, in erster Linie mit Methanol, verethert sind.

Diese Derivate weisen, ebenso wie Papulacandin A und B selbst, eine antimykotische Wirkung auf Fadenpilze und auf hefeartige Pilze, wie insbesondere auf Candida albicans, auf.

Eine spezielle Gruppe von Mono- und Di-ethern des Papulacandins A und B wurde in der Belgischen Patentschrift Nr. 858'492 beschrieben. Diese Verbindungen, die auch eine antibiotische Wirksamkeit gegen Pilze aufweisen und dabei auch als Zwischenprodukte anwendbar sind, sind dadurch gekennzeichnet, dass mindestens eine der phenolischen Hydroxylgruppen in 10- und 12-Stellung durch den Rest $-CH_2K$ verethert ist, in welchem das Symbol K, unter anderen Bedeutungen, auch einen gegebenenfalls substituierten carbocyclischen Kohlenwasserstoff- oder Heterocyclylrest darstellt.

Die vorliegende Erfindung betrifft neuartige N-acylierte 11-Aminoderivate des Papulacandins A und B der Formel I

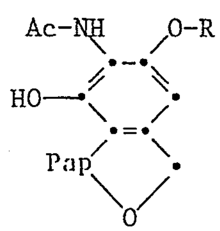

(I) ,

worin Pap den restlichen Teil des Papulacandins A oder B, R Methyl oder Wasserstoff und Ac den Acylrest einer organischen Säure bedeutet, einschliesslich Salze von Verbindungen mit salzbildenden Eigenschaften.

Der Rest Pap ist zweiwertig, wobei beide freien Valenzen vom C-1 ausgehen und dieselbe sterische Orientation wie im Naturstoff der Formel P haben, und umfasst beide Zuckerringe des Papulacandins A oder B (C-1 bis C-6 und C-1' bis C-6') einschliesslich der in der Formel P dargestellten Säurereste $S^1$ der C-18-Säure und $S^2_A$ bzw. $S^2_B$ der für Papulacandin A bzw. B charakteristischen C-10-Säure; in analoger Weise betreffen die Symbole $Pap_A$ und $Pap_B$ spezifisch den restlichen

Teil des Papulacandins A bzw. B. (Sofern dies nicht ausdrücklich angegeben wird, sind unter der allgemeinen Bezeichnung Papulacandin oder Pap infolge ihrer nahen Verwandschaft Verbindungen dieser beiden Grundstrukturen oder ihre Gemische in beliebigem Verhältnis zu verstehen). Im allgemeinen ist $Pap_B$ bevorzugt.

Der durch das Symbol Ac bezeichnete Acylrest einer organischen Säure ist ein solcher, der sich von einer Carbonsäure, einer organischen Sulfonsäure oder einem Kohlensäuremonoderivat ableitet und vorzugsweise höchstens 18 Kohlenstoffatome aufweist.

Der Acylrest einer Carbonsäure in der Bedeutung von Ac kann sich von einer gegebenenfalls substituierten acyclischen, carbocyclischen, carbocyclisch-acyclischen, heterocyclischen oder heterocyclischacyclischen Carbonsäure, insbesondere einer Monocarbonsäure mit höchstens 18 Kohlenstoffatomen ableiten. Beispielsweise sind die folgenden Carbonsäuren zu nennen: Ameisensäure, gegebenenfalls substituierte Alkancarbonsäuren (wie insbesondere die weiter unten hervorgehobenen), halogenierte Niederalkancarbonsäuren (wie die Chloressigsäure, Bromessig- oder α-Bromisovaleriansäure), carbocyclische oder carbocyclisch-acyclische Monocarbonsäuren (wie die Cyclopropan-, Cyclobutan-, Cyclopentan- und Cyclohexan-carbonsäure bzw. die Cyclopropan-, Cyclobutan-, Cyclopentan- oder Cyclohexan-essigsäure oder -propionsäure), aromatische carbocyclische Carbonsäuren (wie Benzoesäure), die einfach oder mehrfach durch Halogene (wie Fluor, Chlor oder Brom) und/oder Hydroxy, Niederalkoxy (wie Methoxy), Niederalkyl (wie Methyl) und Nitro substituiert sein können, Aryl- oder Aryloxy-niederalkancarbonsäuren und deren in der Kette ungesättigte Analoga, welche gegebenenfalls, wie oben für die Benzoesäure angegeben, substituiert sein können (wie Phenylessig- bzw. Phenoxyessigsäure, Phenylpropionsäure und Zimtsäure) und heterocyclische Säuren (wie Furan-2-carbonsäure, 5-tert-Butylfuran-2-carbonsäure, 5-Bromfuran-2-carbonsäure, Thiophen-2-carbonsäure, Nicotin- oder Isonicotinsäure, 4-Pyridinpropionsäure, und gegebenenfalls durch Niederalkylreste substituierte Pyrrol-2- oder -3-carbonsäuren).

Eine dem Acylrest Ac zugrunde liegende Alkancarbonsäure besitzt vorzugsweise nicht mehr als 18 Kohlenstoffatome, wenn sie unsubstituiert ist, und vorzugsweise nicht mehr als 8 Kohlenstoffatome, wenn sie substituiert ist. Die Substituenten sind einerseits Hydroxyl-, Mercapto-, Niederalkylmercapto- (wie Methylmercapto-) Guanidino-, Carboxyl-, veresterte Carboxyl-, Carboxamido- und vor allem primäre Aminogruppen, oder eine an 2 verschiedenen Kohlenstoffatomen gebundene Iminogruppe, andererseits mono- oder bicyclische Hydrocarbyl- oder Heterocyclylreste, wie insbesondere Phenyl, p-Hydroxyphenyl, 1- oder 2-Naphthyl, 2-, 3- oder 4-Pyridyl, 2- oder 3-Indolyl, 2- oder 4-Imidazolyl, 2-, 4- oder 5-Thiazolyl, 2-Thienyl oder 2-Furyl. Die Säure kann einen oder mehrere gleichartige oder verschiedenartige Substitutenten tragen, wobei die gesamte Anzahl der Kohlenstoffatome einschliesslich der kohlenstoffhaltigen Substituenten vorzugsweise höchstens 18 C-Atome beträgt. Besonders bevorzugt sind Acylreste, abgeleitet von einfach verzweigten oder insbesondere geradkettigen unsubstituierten Alkan-(mono oder di)-carbonsäuren, wobei die ersteren höchstens 18, die letzteren höchstens 9 Kohlenstoffatome besitzen, wie von der Essig-, Propion-, Butter-, Isobutter-, Valerian-, Isovalerian-, Capron-, Oenanth-, Undecan-, Laurin-, Myristin-, Palmitin- und Stearinsäure einerseits und der Malon-, Bernstein-, Glutar-, Adipin-, Pimelin- und Korksäure andererseits.

Besonders bevorzugt sind Acylreste abgeleitet von Aminosäuren, vor allem von den in der Natur, insbesondere als Peptid-Bausteine, vorkommenden α-Aminosäuren der L-Reihe und deren nahe verwandten Analogen, wie insbesondere den Enantiomeren der "unnatürlichen" D-Reihe, welche auch in einer geschützten Form vorliegen können, d.h. in einer solchen Form, in welcher die Amino-, Hydroxy- und/oder Mercaptogruppen durch konventionelle, in der Peptidchemie übliche Schutzgruppen substituiert sind. Unter den bevorzugten α-Aminosäuren kommen beispielsweise die folgenden ganz besonders in Betracht:

Glycin, Alanin, Valin, Leucin, Isoleucin, Phenylalanin, Asparaginsäure, Glutaminsäure, Arginin, Lysin und Histidin, ferner β-Alanin, α-Aminobuttersäure, γ-Aminobuttersäure, Norvalin, Isovalin, Norleucin und Ornithin, sowie auch Asparagin, Glutamin, Tyrosin, Tryptophan, Methionin, Threonin und Serin, ferner auch Prolin und Hydroxyprolin, bei denen die α-Aminogruppe mit dem Alkylrest zu einem Ring zusammengeschlossen ist.

Der Acylrest einer organischen Sulfonsäure in der Bedeutung von Ac ist insbesondere derjenige einer aliphatischen oder carbocyclischen, gegebenenfalls ungesättigten bzw. aromatischen Sulfonsäure, vorzugsweise solche, die höchstens 12 Kohlenstoffatome aufweisen. Solche Säuren sind u.a. gegebenenfalls substituierte, z.B. halogenierte, Niederalkansulfonsäuren, Cycloalkansulfonsäuren, worin der Cycloalkylrest mono- oder polycyclisch sein kann, oder gegebenenfalls durch Niederalkyl (z.B. Methyl), Niederalkoxy (z.B. Methoxy), Halogen (z.B. Chlor oder Brom) und/oder Nitro, substituierte Benzolsulfonsäuren. Als typische Beispiele solcher Säuren seien die Trifluormethansulfonsäure, (+)-Campher-10-sulfonsäure, 4-Brom-benzolsulfonsäure und 3-Nitrobenzolsulfonsäure, und insbesondere die Ethansulfonsäure, Benzolsulfonsäure, p-Toluolsulfonsäure und vor allem Methansulfonsäure erwähnt.

Der Acylrest eines Kohlensäuremonoderivats in der Bedeutung von Ac leitet sich insbesondere von einem Kohlensäuremonoester oder Kohlensäuremonoamid ab. Der Acylrest, der sich von einem Kohlensäuremonoester ableitet, ist vorzugsweise ein gegebenenfalls substituiertes Niederalkoxycarbonyl oder Arylniederalkoxycarbonyl. Das erstere kann z.B. durch Halogene, Amino, Carboxyl und/oder verestertes Carboxyl substituiert sein, das letztere kann am Arylrest z.B. Halogen-, Niederalkyl-, Niederalkoxy-, Amino- und/oder Nitro-Substituenten fragen. Beispielsweise sind zu erwähnen: Methoxy-, Ethoxy-, tert-Butoxy-, 2,2,2-Trichlorethoxy- und 2-Jodethoxy-carbonyl, sowie Benzyloxy-, 4-Nitrobenzyloxy-, 4-Brombenzyloxy-, 4-Tolyloxy, 4-Methoxybenzyloxy- und 3,4-Methylendioxybenzyloxy-carbonyl. Solche

Acylreste können sich aber auch von komplizierteren Verbindungen, wie von Hydroxycarbonsäuren, z.B. der Glykol- oder Milchsäure, oder Hydroxyamir.osäuren, wie Hydroxyprolin, Threonin, Allothreonin oder insbesondere Serin, deren Hydroxylgruppe durch Carbonyl zum entsprechenden Kohlensäuremonoesterrest verestert wird, ableiten.

Ein Acylrest, dem ein Kohlensäuremonoamid zugrundeliegt, ist durch die Formel

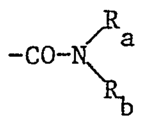

($Ac_u$)

näher charakterisiert, worin $R_a$ und $R_b$ unabhängig je für ein Wasserstoffatom oder einen gegebenenfalls substituierten Niederalkyl- oder Niederalkanoyl-rest mit höchstens 7 Kohlenstoffatomen steht, wobei die beiden Reste $R_a$ und $R_b$ durch eine Kohlenstoff-Kohlenstoff-Bindung oder ein Sauerstoff-, Schwefel- oder gegebenenfalls substituiertes Stickstoffatom untereinander gebunden sein und zusammen mit dem Stickstoffatom der Aminogruppe einen stickstoffhaltigen Heterocyclischen Ring bilden können. Die Substituenten der Niederalkyle und -alkanoyle sind einerseits Hydroxyl-, Oxo-, Mercapto- (wie Methylmercapto-) und Amino-gruppen (unter den letzteren vorzugsweise primäre Aminogruppen und Mono- oder Di-niederalkylaminogruppen mit insgesamt höchstens 8 C-Atomen, z.B. Methylamino, Ethylamino, Dimethylamino und Diethylamino), andererseits monocyclische Hydrocarbyl- oder Heterocyclylreste, insbesondere Cyclohexyl, Phenyl, 4-Pyridyl, 2- oder 4-Imidazolyl, 2-, 4- oder 5-Thiazolyl, 2-Thienyl oder 2-Furyl. Vorzugsweise kann nur einer der Reste $R_a$ und $R_b$ für ein Niederalkanoyl stehen.

Ein bevorzugter Rest $Ac_u$ ist beispielsweise Carbamoyl, Niederalkylcarbamoyl (wie Methylcarbamoyl und Ethylcarbamoyl) und Diniederalkylcarbamoyl (wie Dimethylcarbamoyl, Methyl-ethyl-carbamoyl, Diethylcarbamoyl, Dipropylcarbamoyl oder Dibutylcarbamoyl), ferner auch Niederalkanoylcarbamoyl (wie Acetylcarbamoyl oder Formylcarbamoyl) oder Niederalkanoyl-niederalkylcarbamoyl (wie N-Formyl-N-methylcarbamoyl, N-Acetyl-N-methylcarbamoyl, N-Formyl-N-ethylcarbamoyl oder N-Acetyl-N-ethylcarbamoyl). - Ein Rest $Ac_u$, in welchem die

Reste $R_a$ und $R_b$ mittels einer C-C-Bindung, vorzugsweise einer einfachen C-C-Bindung, verknüpft sind, ist beispielsweise 1-Azetidinylcarbonyl, 1-Perhydroazepinylcarbonyl, 1-Perhydroazocinylcarbonyl und insbesondere 1-Pyrrolidinylcarbonyl und Piperidinocarbonyl, welche auch die oben genannten Substituenten beider Art oder auch substituierte oder vorzugsweise unsubstituierte Niederalkylreste tragen können, wobei die Gesamtzahl der Kohlenstoffatome eines solchen Acylrestes höchstens 15 beträgt. Unter derartigen Resten sind die folgenden beispielsweise zu erwähnen: ein durch Hydroxyl, Carboxyl, verestertes Carboxyl oder Oxo substituiertes 1-Pyrrolidinylcarbonyl, ein durch Formyl, Carboxyl, Niederalkoxycarbonyl, Carbamoyl oder Oxo substituiertes Piperidinocarbonyl, wobei der Substituent sich vorzugsweise in 4-Stellung oder, wenn er Oxo ist, auch in 2-Stellung befindet; ein Niederalkyl-, 1-Hydroxyniederalkyl- oder 2-Hydroxyniederalkyl-piperidinocarbonyl, wobei der Rest sich vorzugsweise in der 4-Stellung befindet, sowie 1-(2-Oxoperhydroazepinyl)carbonyl.

Ein Rest $Ac_u$, in welchem der heterocyclische Ring durch die Verknüpfung der Reste $R_a$ und $R_b$ mittels eines Sauerstoff- oder Schwefel(II)-atoms gebildet ist, ist beispielsweise Morpholinocarbonyl, Thiomorpholinocarbonyl und 3-Thiazolidinylcarbonyl.

Im Rest $Ac_u$, in welchem der heterocyclische Ring durch die Verknüpfung der Reste $R_a$ und $R_b$ mittels eines gegebenenfalls substituierten Stickstoffatoms gebildet wird, ist dieses Stickstoffatom durch die Partialformel

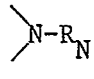

charakterisiert, worin $R_N$ Wasserstoff, Formyl, Carboxyl, Niederalkoxycarbonyl (wie Methoxy- und Ethoxy-carbonyl), Carbamoyl, und insbesondere einen höchstens 8 C-Atome aufweisenden acyclischen oder monocyclischen Kohlenwasserstoff, z.B. Niederalkyl (insbesondere Methyl), Niederalkenyl (wie Allyl), Niederalkynyl (wie Propargyl), Phenyl, Phenylniederalkyl (insbesondere Benzyl), sowie auch Hydroxyniederalkyl (insbesondere 2-Hydroxyethyl) bedeuten kann. Ein derartiger Rest $Ac_ü$ ist insbesondere ein 1-Morpholinylcarbonyl, 1-Pyrimidinylcarbonyl und 1-Imidazolidinylcarbonyl, die sowohl an ihren Kohlenstoffatomen wie auch am anderen Stickstoffatom in der oben angegebenen Weise substituiert sein können. Unter den Substituenten der Kohlenstoffatome ist die Oxogruppe besonders zu erwähnen, insbesondere wenn sie am C-Atom neben dem zentralen Stickstoffatom steht. Besonders bevorzugte Acylreste dieses Typs sind charakterisiert durch die Formel $Ac_t$

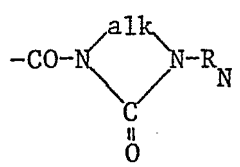 $(Ac_t)$ ,

worin $R_N$ die obgenannte Bedeutung hat und alk einen Alkylenrest mit 2-8 C-Atomen bedeutet, der die beiden Stickstoffatome voneinander durch mindestens 2 und höchstens 4 C-Atome trennt. Vorzugsweise bedeutet $R_N$ Wasserstoff, Niederalkyl mit 1-4 C-Atomen, insbesondere Methyl, oder Benzyl; das Symbol alk steht vorzugsweise für Tetramethylen, Trimethylen oder vor allem für Ethylen. Unter den Acylresten der Formel $Ac_t$ sind besonders zu erwähnen: 1-(2-Oxoperhydro-1,3-diazepinyl)carbonyl, 1-(2-Oxopyrimidinyl)-carbonyl, 1-(3-Methyl-2-oxopyrimidinyl)-carbonyl, 1-(3-Benzyl-2-oxoimidazolidinyl)-carbonyl, 1-(3-Methyl-2-oxoimidazolidinyl)-carbonyl und ganz speziell das 1-(2-Oxoimidazolidinyl)-carbonyl.

Unter den erfindungsgemässen Verbindungen der Formel I sind diejenigen bevorzugt, worin Pap für $Pap_B$, R für Methyl oder vorzugsweise Wasserstoff und Ac für ein Niederalkanoyl, in erster Linie Acetyl steht.

Unter den erfindungsgemässen Verbindungen der Formel I sind auch diejenigen bevorzugt, worin Pap für $Pap_B$, R für Methyl oder vorzugsweise Wasserstoff und Ac für den Acylrest einer in der Natur vorkommenden α-Aminosäure, insbesondere Glycyl, Alanyl, α- oder β-Aspartyl, Asparaginyl, α- oder γ-Glutamyl oder Glutaminyl steht.

Unter den erfindungsgemässen Verbindungen der Formel I sind diejenigen besonders bevorzugt, worin Pap für $Pap_B$, R für Methyl oder vorzugsweise Wasserstoff und Ac für den Acylrest einer gegebenenfalls durch Halogen, Nitro, Methoxy oder Methyl substituierten Benzolsulfonsäure oder Niederalkansulfonsäure, vor allem der Methansulfonsäure, steht.

Unter den erfindungsgemässen Verbindungen der Formel I sind diejenigen ganz besonders bevorzugt, worin Pap für $Pap_B$, R für Methyl oder vorzugsweise Wasserstoff und Ac für den oben definierten Acylrest $Ac_t$ steht, vor allem einen solchen, worin alk Ethylen und $R_N$ Wasserstoff, Methyl oder Benzyl bedeutet.

Ganz besonders bevorzugt sind Verbindungen der oben stehenden Formel I, in welchen Ac für einen Acylrest gemäss der Durchführungsbeispiele steht, oder welche das Grundgerüst eines der in den Beispielen gezeigten Papulacandin-Derivate haben. Vor allem sind dann die in den Beispielen gezeigten N-acylierten Papulacandin-Derivate bevorzugt.

Diejenigen der oben allgemein oder als bevorzugt charakterisierten Verbindungen der Formel I, die eine freie Carboxylgruppe enthalten, können auch als Salze vorliegen, z.B. Natrium-, Kalium-, Calcium- oder Magnesiumsalze, oder auch Ammoniumsalze abgeleitet von Ammoniak oder einer stickstoffhaltigen organischen Base, welche physiologisch verträgliche Salze mit Verbindungen der Formel I zu bilden vermögen. Diejenigen der oben allgemein oder als bevorzugt charakterisierten Verbindungen der Formel I, die eine freie Aminogruppe enthalten, können auch in Form von Salzen, und zwar von Säureadditionssalzen, vorliegen. Als Säureadditionssalze kommen physiologisch verträgliche Salze mit üblichen therapeutisch anwendbaren Säuren besonders in Betracht; von den anorganischen Säuren sind die Halogenwasserstoffsäuren, wie die Chlorwasserstoffsäure, aber auch Schwefelsäure und Phosphor- bzw. Pyrophosphorsäure zu nennen, von den organischen Säuren sind es in erster Linie die Sulfonsäuren, z.B. die Benzol- oder p-Toluol-sulfonsäure oder Niederalkansulfonsäuren, wie Methansulfonsäure, ferner auch Carbonsäuren, wie Essigsäure, Milchsäure, Palmitin- und Stearinsäure, Aepfelsäure, Weinsäure, Ascorbinsäure und Zitronensäure. - Verbindungen, die sowohl eine Aminogruppe wie eine freie Carboxylgruppe haben, können auch als innere Salze vorliegen.

Wenn nicht anders angegeben, beziehen sich die Namen der Aminosäuren bzw. ihre Kurzbezeichnungen auf α-Aminosäuren der L-Reihe, welche in der Natur vorkommen.

Wenn nicht anders angegeben, bezeichnet der Begriff "nieder", wo immer er im Zusammenhang mit einem organischen Rest oder Verbindung vorkommt, einen solchen Rest oder Verbindung mit höchstens 7, vorzugsweise aber mit höchstens 4 Kohlenstoffatomen.

Die neuen erfindungsgemässen Papulacandin-Verbindungen weisen eine antibiotische Wirkung auf, die im Grundcharakter der Wirkung anderer Papulacandine ähnlich ist. Sie können deshalb in ähnlichen therapeutischen Indikationen wie diese, z.B. insbesondere zur Bekämpfung von Infektionen durch Pilze, insbesondere durch Candida albicans, verwendet werden. Sie können auch als wertvolle Zwischenprodukte zur Herstellung anderer therapeutisch wertvoller Verbindungen dienen, z.B. solcher mit einem weiter modifizierten Acylrest Ac.

Die erfindungsgemässen Papulacandin-Derivate der Formel I werden unter Anwendung von konventionellen, an sich bekannten Herstellungsverfahren der präparativen organischen Chemie erhalten. So werden sie

beispielsweise hergestellt, indem man ein entsprechendes 11-Aminopapulacandin-Derivat unter vorübergehendem Schutz vorhandener freier Hydroxylgruppe in 12-Stellung acyliert und wenn erwünscht, ein erhaltenes Produkt mit salzbildenden Eigenschaften als Salz isoliert. Falls im Acylierungsmittel freie funktionelle Gruppen, wie Hydroxyl-, Carboxyl- und vor allem Aminogruppen, vorkommen, die an der Acylierungsreaktion nicht teilnehmen, so können sie nötigenfalls auch vorübergehend geschützt und nach der Hauptreaktion freigesetzt werden. Das erfindungsgemässe Verfahren ist insbesondere dadurch gekennzeichnet, dass man ein 11-Aminopapulacandin-Derivat der Formel

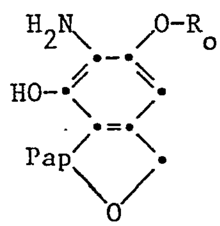

(II) ,

worin Pap die oben angegebene Bedeutung hat und $R_o$ für Methyl oder eine Hydroxyl-Schutzgruppe Y steht, mit einer organischen Säure der Formel

AcOH (III)

oder einem reaktionsfähigen Derivat davon, in welchen Ac die oben genannte Bedeutung hat, gegebenenfalls unter vorübergehendem Schutz von im Reste Ac vorhandenen Amino-, Hydroxyl- und Carboxyl-Gruppen durch entsprechende Schutzgruppen X, Y bzw. W, umsetzt und eine im Rest $R_o$ vorliegende Schutzgruppe Y, und gewünschtenfalls auch im Rest Ac vorliegende Schutzgruppen, abspaltet und, wenn erwünscht, eine erhaltene Verbindung mit salzbildenden Eigenschaften in ihr Salz, oder ein erhaltenes Salz in die entsprechende freie Verbindung umwandelt.

Ein reaktionsfähiges Derivat der Säure AcOH ist beispielsweise ein Anhydrid, insbesondere ein symmetrisches Anhydrid der Formel Ac-O-Ac oder ein cyclisches Anhydrid einer Dicarbonsäure, wie Succinanhydrid oder Glutaranhydrid, oder aber ein gemischtes Anhydrid mit einer anderen organischen Säure, z.B. mit Trifluoressigsäure, oder insbesondere mit einer anorganischen Säure, z.B. ein Säureazid oder Säurehalogenid, vor allem Säurechlorid. Ein reaktionsfähiges Säurederivat ist vorzugsweise ein aktivierter Ester, z.B. ein solcher, in welchem die Säure AcOH mit 2,4,5-Trichlorphenol, Pentachlorphenol, Pentafluorphenol, 2-Nitrophenol oder insbesondere 4-Nitrophenol, oder mit einer N-Hydroxyverbindung, wie N-Hydroxysuccinimid, 1-Hydroxybenzotriazol oder N-Hydroxypiperidin, oder aber mit einem N,N'-disubstituierten Isoharnstoff, wie insbesondere N,N'-Dicyclohexylisoharnstoff, oder einer ähnlichen allgemein bekannten Aktivierungskomponente verestert ist.

Als Acylierungsmittel sind im allgemeinnen Aktivester deshalb vorteilhaft, weil sie Aminogruppen bevorzugt vor Hydroxylgruppen acylieren und somit den Schutz von Hydroxylgruppen praktisch überflüssig machen. Bei Dicarbonsäuren sind jedoch cyclische Anhydride bevorzugt, sofern sie vorhanden sind. Um eine unerwünschte O-Acylierung zu vermeiden, verwendet man vornehmlich nur ein Aequivalent des Acylierungsmittels bezogen auf den Ausgangsstoff der Formel II.

Die Acylierung erfolgt in an sich bekannter Weise, vorzugsweise in üblichen Lösungsmitteln, beispielsweise Dioxan, Tetrahydrofuran, Acetonitril, Pyridin, Dimethylformamid, Dimethylacetamid, Dimethylsulfoxid, N-Methylpyrrolidon, Hexamethylphosphortriamid, sowie Chloroform und Methylenchlorid, und in zweckmässigen Gemischen davon. Man kann auch mit einem Zusatz einer organischen Base, z.B. eines quaternären oder vor allem tertiären Amins, wie Triethylamin, N-Ethylmorpholin oder N-Methylpiperidin, arbeiten, um die zu acylierende

Aminogruppe in entprotonisierter Form zu halten. Die Reaktionstemperatur beträgt üblicherweise -20° bis +50°C, vorzugsweise etwa 0°C bis Raumtemperatur. - Wenn man mit überschüssigen Acylierungsmitteln, wie einem symmetrischen Anhydrid, arbeitet, ist es vorteilhaft, einen Niederalkanol, insbesondere Methanol oder Ethanol, als Lösungsmittel zu verwenden, die Reaktionstemperatur zwischen etwa -10 bis +25° zu halten und die Reaktion nach erfolgter N-Acylierung (die ohnehin mit wesentlich höherer Reaktionsgeschwindigkeit als die O-Acylierung vor sich geht) abzubrechen; der geeignete Zeitpunkt kann mit üblichen analytischen Methoden, z.B. mittels Dünnschichtchromatographie, einfach ermittelt werden.

Bei einer besonderen Durchführungsvariante, die man vornehmlich bei Aminosäuren verwenden kann, wird mit einer Aminosäure mit aktivierter terminaler Carboxylgruppe und geschützter α-Aminogruppe umgesetzt. Die Carboxylgruppe kann dabei direkt in situ durch die Reaktion einer freien Säure mit einem Carbodiimid, wie N,N'-Dicyclohexylcarbodiimid, gegebenenfalls unter Zusatz von N-Hydroxysuccinimid, einem unsubstituierten oder z.B. durch Halogen, Methyl oder Methoxy substituierten 1-Hydroxybenzotriazol oder 4-Hydroxybenzo-1,2,3-triazin-3-oxid (u.a., vgl. DT 2 202 613), oder insbesondere vom N-Hydroxy-5-norbornen-2,3-dicarboximid, oder mit N,N'-Carbonyldiimidazol aktiviert werden. - Die Arbeitsbedingungen dieser und analoger Acylierungsmethoden sind insbesondere als ein allgemeines Verfahren zur Synthese von Peptiden sehr eingehend ausgearbeitet, vgl. Houben-Weyl: Methoden der organischen Chemie; 4. Auflage, Band 15/I und II, E. Wünsch (Herausgeber): Synthese von Peptiden (Georg Thieme Verlag, Stuttgart; 1974). Vornehmlich wird die Acylierungsreaktion in der weiter unten durch die Beispiele illustrierten Weise durchgeführt.

Auch die zu verwendenden Schutzgruppen X, Y und W sind insbesondere von der Peptidchemie, vgl. z.B. das oben zitierte Nachschlagewerk,

allgemein bekannt. Die engere Auswahl der Schutzgruppen richtet sich nach dem spezifischen Zweck, wobei man insbesondere die speziellen Eigenschaften des Papulacandin-Rests (Ester und Ether-Bindungen der Zuckerreste, Doppelbindungen der Acylreste $S^1$ und $S^2$) berücksichtigen muss. Bei mehreren zu schützenden funktionellen Gruppen sind zweckmässige Kombinationen zu wählen. Vorzugsweise werden z.B. gleichartige oder noch besser gleiche Schutzgruppen sowohl im Rest $R_o$ wie auch Ac verwendet und im Anschluss an die Acylierungsreaktion gleichzeitig abgespalten.

Als Amino-Schutzgruppe X kann man vornehmlich reduktiv abspaltbare Amino-Schutzgruppen verwenden, z.B. insbesondere die vom Benzyloxycarbonyl-Typ, worin die Benzyloxycarbonyl-Gruppe im aromatischen Teil durch Halogenatome, Niederalkoxygruppen und/oder Niederalkylreste und vor allem Nitrogruppen substituiert ist, wie die p-Chlor- und p-Brombenzyloxycarbonyl-, p-Methoxybenzyloxycarbonyl-, p-Tolyloxycarbonyl- und vor allem p-Nitrobenzyloxycarbonyl-Gruppe, oder aber die Isonicotinyloxycarbonylgruppe. Eine vorteilhafte Amino-Schutzgruppe X ist eine Ethoxycarbonylgruppe, die in β-Stellung eine mit drei Kohlenwasserstoffresten substituierte Silylgruppe, wie Triphenylsilyl-, Dimethyl-butyl-silyl- oder vor allem Trimethylsilylgruppe, trägt. Eine solche β-(Trihydrocarbylsilyl)-ethoxycarbonylgruppe, wie eine β-(Triniederalkylsilyl)-ethoxycarbonyl-, z.B. insbesondere die β-(Trimethylsilyl)-ethoxycarbonylgruppe, bildet mit der zu schützenden Aminogruppe eine entsprechende β-Trihydrocarbylsilylethoxycarbonylaminogruppe (z.B. die β-Trimethylsilylethoxycarbonylaminogruppe), welche sich nur unter ganz spezifischen, sehr milden Bedingungen durch Einwirkung von Fluoridionen abspalten lässt. In dieser Beziehung verhält sie sich analog wie die weiter unten als Carboxyl-Schutzgruppe beschriebene β-Silylethylestergruppe. (Diese Aehnlichkeit ist bei der Synthese von besonderem Vorteil, wenn im Acylrest Ac beide diese Schutzgruppen gleichzeitig abgespalten werden sollen). Weitere Einzelheiten sind weiter unten beim Schutz

der Carboxylgruppe als β-Silylethylester angegeben. Unter Umständen kann man auch acidolytisch abspaltbare Gruppen, wie die tert-Butoxycarbonyl-Gruppe und analoge Gruppen sowie auch die des Aralkyl-Typs, wie Benzhydryl, Di-(4-methoxy)-benzhydryl und Triphenylmethyl (Trityl), oder bestimmte Aralkoxycarbonyl-Gruppen des 2-(p-Biphenylyl)-2-propyloxycarbonyl-Typs, die in der Schweizer Patentschrift 509 266 beschrieben sind, verwenden.

Als Hydroxyl-Schutzgruppe Y können mit Vorteil reduktiv abspaltbare Gruppen verwendet werden, vgl. das oben zitierte Werk (Houben -Weyl), ferner auch acidolytisch abspaltbare Gruppen, wie 2-Tetrahydropyranyl und tert-Butyl. Bevorzugte reduktiv abspaltbare Hydroxyl-Schutzgruppen sind z.B. Benzylgruppen, die im aromatischen Teil durch Halogen, Niederalkyl, Niederalkoxy und/oder vor allem Nitro substituiert sind, insbesondere die 4-Nitrobenzylgruppe. Weiter können auch Acylgruppen, die unter schwach basischen Bedingungen abspaltbar sind, wie Formyl oder Trifluoracetyl, verwendet werden.

Zum Schutz von Carboxylgruppen werden diese vorzugsweise verestert. Die dazu gebräuchlichen Carboxyl-Schutzgruppen W sind allgemein bekannt, vgl. z.B. das oben zitierte Nachschlagewerk (Houben-Weyl). Zur Veresterung geeignet sind z.B. Alkohole, die acidolytisch abspaltbare Reste ergeben, wie Cyanmethylalkohol, Benzoylmethylalkohol oder tert-Butylalkohol, besonders aber solche, die reduktiv abspaltbare Reste ergeben, wie 2,2,2-Trichlorethanol und vor allem 4-Nitrobenzylalkohol oder auch Isonicotinylalkohol. Eine besonders vorteilhafte Kategorie der substituierten Alkanole sind Ethylalkohole, die in β-Stellung eine trisubstituierte Silylgruppe, wie Triphenylsilyl-, Dimethyl-butyl-silyl- oder vor allem Trimethylsilylgruppe, tragen. Wie z.B. im belgischen Patent Nr. 851.576 beschrieben ist, eignen sich diese Alkohole zum Schutze der Carboxylgruppen deshalb besonders gut, weil die entsprechenden β-Silylethylester, z.B.

β-(Trimethylsilyl)-ethylester, zwar die Stabilität üblicher Alkylester besitzen, sich jedoch unter milden Bedingungen durch Einwirkung von Fluoridionen unter Erhaltung anderer veresterter Carboxylgruppen, z.B. der im Rest Pap, selektiv abspalten lassen.

Die erfindungsgemässe nachträgliche Abspaltung vorhandener Schutzgruppen richtet sich nach deren Art und erfolgt jeweils in einer an sich bekannten, konventionellen Weise, jedoch unter Berücksichtigung der Eigenschaften des Restes Pap. Dabei ist die Abspaltung einer vorhandenen Hydroxylschutzgruppe Y im Rest $R_o$ eine obligatorische Massnahme; dagegen wird eine Schutzgruppe X, Y und/oder W im Ac-Rest nur gewünschtenfalls abgespalten. Würden die Schutzgruppen X, Y und W so gewählt, dass sie unter ähnlichen Bedingungen abspaltbar sind (besonders bevorzugt sind dabei die bereits hervorgehobenen reduktiv abspaltbaren Gruppen), dann erfolgt die Abspaltung aller dieser Schutzgruppen vorteilhaft in einer einzigen Operation; in besonderen Fällen können jedoch auch Gruppen verschiedener Art verwendet werden und jede einzeln abgespalten werden.

Die reduktiv abspaltbaren Gruppen, insbesondere solche, die halogenierte Niederalkylreste (z.B. 2,2,2-Trichlorethylreste), Isonicotinylreste (z.B. Isonicotinyloxycarbonyl) und insbesondere substituierte Benzylreste, vor allem 4-Nitrobenzylreste aller Art, enthalten, werden vorzugsweise durch Zink-Reduktion, üblicherweise in Anwesenheit einer Säure, vorzugsweise der Essigsäure, und gegebenenfalls unter Zugabe eines inerten organischen Lösungsmittels, üblicherweise bei Raumtemperatur, entfernt. Die Abspaltung einer Schutzgruppe durch die saure Hydrolyse (Acidolyse) wird bei Gruppen vom tert-Butyl-Typ mittels Fluorwasserstoff oder Trifluoressigsäure, bei säureempfindlichen Schutzgruppen vornehmlich mittels einer niederaliphatischen Carbonsäure, wie Ameisensäure und/oder Essigsäure, in Anwesenheit von Wasser und gegebenenfalls von einem polyhalogenierten Niederalkanol oder Niederalkanon, wie 1,1,1,3,3,3-Hexafluorpropan-2-ol oder

Hexafluoraceton, durchgeführt. In dieser Weise kann man z.B. eine N-Tritylgruppe mit einer organischen Säure, wie Ameisensäure, Essigsäure, Chloressigsäure oder Trifluoressigsäure, in wässrigem oder absolutem Trifluorethanol als Lösungsmittel (vgl. deutsche Offenlegungsschrift DT 2 346 147) oder mit wässeriger Essigsäure; die tert-Butyloxycarbonylgruppe mit Trifluoressigsäure oder Salzsäure; die 2-(p-Biphenylyl)-isopropyloxycarbonylgruppe mit wässeriger Essigsäure oder z.B. mit einem Gemisch von Eisessig, Ameisensäure (82,8%ig) und Wasser (7:1:2) oder nach dem Verfahren der DT 2 346 147, abspalten. Die β-Silylethylestergruppen werden vorzugsweise mit Fluoridionen-abgebenden Reagentien, z.B. Fluoriden quaternärer organischer Basen, wie Tetraethylammoniumfluorid, abgespalten. - Schutzgruppen, die basen-labil sind, z.B. die Formyl und Trifluoracetylgruppen, können schonend durch eine kurze Einwirkung von einer wässrigen Natrium- oder Kaliumbicarbonat-Lösung oder, vorzugsweise, wässrigem Ammoniak in einem organischen Lösungsmittel, üblicherweise bei Raumtemperatur, entfernt werden. - Vorzugsweise wird unter den Reaktionsbedingungen der Beispiele, oder analogen Bedingungen, gearbeitet.

Diejenigen erfindungsgemässen Endstoffe, die basische Gruppen enthalten, werden, je nach der Art der Isolierung, als Basen oder als Säureadditionssalze erhalten; analogerweise können Endstoffe mit sauren Gruppen auch in Form von Salzen vorliegen. Beide Formen sind jeweils ineinander in bekannter Weise überführbar. Aus den Säureadditionssalzen können in an sich bekannter Weise die Basen gewonnen werden. Von letzteren wiederum lassen sich durch Umsetzen mit Säuren, z.B. mit solchen, die die oben genannten Salze bilden, Säureadditionssalze, insbesondere therapeutisch anwendbare Säureadditionssalze, gewinnen. In ähnlicher Beziehung stehen auch Säuren und ihre Salze zueinander. Verbindungen, die sowohl eine freie Carboxylgruppe und eine basische Gruppe haben, können als innere Salze vorliegen, diese werden z.B. durch die Einstellung des isoelektrischen Punktes gewonnen.

Infolge der engen Beziehung zwischen der freien Form und einer Salz-Form sind im vorausgegangenen und nachfolgend unter allen in Frage kommenden freien Verbindungen oder ihren Salzen sinn- und zweckgemäss gegebenenfalls auch die entsprechenden Salze bzw. freien Verbindungen zu verstehen.

Die Erfindung betrifft auch diejenigen Ausführungsformen der Verfahren, bei denen man von einer auf beliebiger Verfahrensstufe als Zwischenprodukt erhältlichen Verbindung ausgeht und die fehlenden Verfahrensschritte ausführt, oder wobei ein Ausgangsstoff unter den Reaktionsbedingungen gebildet oder in Form eines Derivats davon, zum Beispiel eines Salzes, verwendet wird.

Beim Verfahren der vorliegenden Erfindung werden vorzugsweise solche Ausgangsstoffe verwendet, welche zu den eingangs als besonders wertvoll geschilderten Verbindungen führen.

Die Ausgangsstoffe der Formel III, d.h. die Säuren der Formel AcOH und ihre reaktionsfähigen Derivate, sind bekannt oder, sofern sie unbekannt sind, nach konventionellen synthetischen Verfahren einfach zugänglich.

Die für das erfindungsgemässe Verfahren als Ausgangsstoff verwendeten 11-Aminopapulacandin-Derivate der Formel II

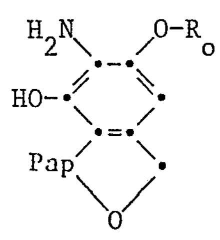

(II)

worin Pap den restlichen Teil des Papulacandins A oder B und $R_o$ Methyl oder eine Hydroxyl-Schutzgruppe Y bedeutet, sowie ihre Säureadditionssalze, sind neu und bilden auch Gegenstand der vorliegenden Erfindung.

Der Rest Pap ist vorzugsweise der Rest $Pap_B$ des Papulacandins B. Die Hydroxyl-Schutzgruppe Y ist vorzugsweise eine reduktiv abspaltbare Hydroxyl-Schutzgruppe, vorzugsweise die Isonicotinyl- oder vor allem die 4-Nitrobenzyl-Gruppe. Bevorzugt als $R_o$ ist jedoch Methyl.

Verbindungen der Formel II, insbesondere diejenigen, worin $R_o$ für Methyl steht, zeichnen sich durch analoge antibiotische Eigenschaften wie die entsprechenden Acylamino-Derivate der Formel I aus, und können zu analogen therapeutischen Zwecken wie jene verwendet werden.

Die Verbindungen der Formel II werden erfindungsgemäss dadurch hergestellt, dass man ein Papulacandin-Derivat der Formel IV

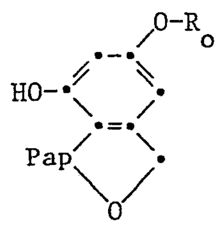

(IV)

worin Pap und $R_o$ die oben genannten Bedeutungen haben, nacheinander a) mit einem Arylseleninsäureanhydrid und Hexaniederalkyldisilazan umsetzt und b) mit einem Reduktionsmittel behandelt.

Das Arylseleninsäureanhydrid ist charakterisiert durch die Formel Ar-(Se=O)-O-(Se=O)-Ar (V), worin Ar einen höchstens 12 C-Atome aufweisenden Arylrest, insbesondere Phenylrest, bedeutet; das Hexaniederalkyldisilazan hat die Formel $(Me_3Si)_2NH$ (VI), worin Me einen Niederalkylrest mit 1-4 C-Atomen, insbesondere Methyl, darstellt. Die Reaktionsbedingungen wurden bereits an Beispielen von einfacheren Phenolen beschrieben, vgl. D.H.R. Barton et al. J. Chem. Soc., Chemical Communications , 1977, 147. Vornehmlich wird die Reaktion mit einem kleineren Ueberschuss von etwa 1,2-1,5 Moläquivalent beider Reagentien (vorzugsweise Phenylseleninsäureanhydrid und Hexamethyldisilazan) durchgeführt, wobei man in einem inerten organischen Lösungsmittel, wie einem aromatischen Kohlenwasserstoff (z.B. Benzol oder Toluol), chloriertem Niederalkan (z.B. Methylenchlorid oder

Chloroform) oder einem aliphatischen oder cyclischen Ether (z.B. Diethylether oder 1,2-Dimethoxyethan, bzw. Tetrahydrofuran oder Dioxan) bei Temperaturen von -10 bis +30°, vorzugsweise bei 0° bis Raumtemperatur, arbeitet.

Die zweite Stufe, d.h. die Reduktion des als Zwischenprodukt erhältlichen Arylselenoimins der allgemeinen Formel

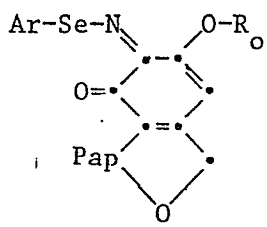

(VII) ,

worin Ar, Pap und $R_o$ die oben genannten Bedeutungen haben, erfolgt unter gleichzeitiger Abspaltung der Arylselenogruppe und führt zur Bildung der 11-Aminogruppe und Regenerierung der 10-Hydroxylgruppe. Die Reduktion wird vorzugsweise mit Schwefelwasserstoff oder einem organischen Substitutionsderivat davon, wie einem aliphatischen oder aromatischen Thiol (z.B. insbesondere Phenylthiol) bei einer Temperatur von etwa -10° bis etwa +30°, vorzugsweise zwischen 0° und Raumtemperatur, durchgeführt, als Reaktionsmedium verwendet man übliche inerte organische Lösungsmittel, z.B. die bei der ersten Stufe genannten, insbesondere Methylenchlorid oder Chloroform. Vorzugsweise, insbesondere bei der Anwendung von Schwefelwasserstoff, reduziert man in der Gegenwart einer organischen Base, insbesondere eines tertiären Amins, z.B. Triethylamin, N-Methylpyrrolidin, N-Ethylpiperidin oder N,N'-Dimethylpiperazin. Vornehmlich erfolgen beide Reaktionen in der in den Beispielen illustrierten Weise.

Die als Ausgangsstoffe verwendbaren Papulacandin-12-Derivate der oben definierten Formel IV sind bekannt (vgl. die deutsche Offenlegungsschrift 2'609'611 und die Belgische Patentschrift 858'492) oder gemäss bekannten Verfahren dieser Schriften zugänglich. Die übrigen

Reagentien sind auch bekannt oder in bekannter Weise zugänglich.

Je nach Arbeitsweise erhält man die Verbindungen der Formel II in Form von Basen oder Säureadditionssalzen. Aus den letzteren können in an sich bekannter Weise die Basen gewonnen werden. Von diesen wiederum lassen sich durch Umsetzten mit Säuren entsprechende Salze, insbesondere therapeutisch anwendbare Säureadditionssalze, gewinnen. Infolge der engen Beziehung zwischen den neuen Verbindungen in freier Form und in Form ihrer Salze sind im vorausgegangenen und nachfolgend unter den freien Verbindungen oder ihren Salzen sinn- und zweckgemäss gegebenenfalls auch die entsprechenden Salze bzw. freien Verbindungen zu verstehen.

Die erfindungsgemässen Verbindungen zeichnen sich durch ihre wertvollen pharmakologischen Eigenschaften, und zwar durch ihre antibiotische, insbesondere antimykotische Wirksamkeit, aus, wie sie sich einerseits in vitro gegen verschiedene Laborstämme und klinische Isolate von Candida albicans (im getesteten Konzentrationsbereich von 0,1 bis 64 $\mu$g/ml), sowie gegen Aspergillus fumigatus, Sporotrichum Schenckii, Trichophyton mentagrophytes und Microsporum canis (im getesteten Konzentrationsbereich von 1 bis 128 $\mu$g/ml), andererseits in vivo (Maus) gegen akute Septicaemie durch Candida albicans (im getesteten Dosisbereich von 13 bis 90 mg/kg s.c.) nachweisen lassen. Besonders hervorzuheben als Wirkstoffe sind 11-Amino-12-O-methylpapulacandin B, 11-Acetamino-12-O-methyl-Papulacandin B, 11-Mesylamino-Papulacandin B, 11-Glycylamino-Papulacandin B, 12-O-Methyl-11-(3-O-L-seryl)-carbonylamino-Papulacandin B und ganz speziell das 11-(2-Oxoimidazolidincarboxamido)-Papulacandin B, welches bei subkutaner Applikation in Maus eine deutliche Hemmwirkung auf die Candida-Septicaemie bei ED = 13 mg/kg aufweist.

In Anlehnung an diese günstigen Eigenschaften umfasst die Erfindung auch die Verwendung der erfindungsgemässen neuen Papulacandin-Derivate,

allein oder in Kombination mit einem anderen Antibiotikum oder Chemotherapeutikum, als ein Mittel zur Bekämpfung von Infektionen, die durch Pilze und Fungi imperfecti, z.B. die genannten, hervorgerufen werden, und zwar sowohl am tierischen oder menschlichen Körper, d.h. als Heilmittel, wie auch ausserhalb des menschlichen bzw. tierischen Körpers, z.B. als Desinfektionsmittel. Bei der Verwendung als Heilmittel, und zwar sowohl zur lokalen wie auch systemischen Behandlung, wird der erfindungsgemässe Wirkstoff vorzugsweise in Form eines pharmazeutischen Präparats zusammen mit mindestens einem konventionellen pharmazeutischen Träger oder Hilfsstoff einem Warmblüter, vor allem Menschen, verabreicht. Diese pharmazeutischen Präparate gehören ebenfalls zum Gegenstand der Erfindung.

Zwecks Herstellung pharmazeutischer Präparate kann jede einzelne der erfindungsgemässen Verbindungen, vor allem eine der besonders hervorgehobenen, mit einem für die topische, enterale oder parenterale Applikation geeigneten anorganischen oder organischen Trägermaterial vermischt werden. Für dasselbe kommen solche Stoffe in Betracht, die mit der neuen Verbindung nicht reagieren, wie z.B. Gelatine, Milchzucker, Stärke, Magnesiumstearat, pflanzliche Oele, Benzylalkohol, oder andere Arzneimittelträger. Die pharmazeutischen Präparate können z.B. als Tabletten, Dragées, Pulver, Suppositorien, oder in flüssiger Form als Lösungen, Suspensionen, Emulsionen, Cremen oder Salben vorliegen. Gegebenenfalls sind sie sterilisiert und/oder enthalten Hilfsstoffe, wie Konservierungsmittel, Stabilisierungs-, Netz- oder Emulgiermittel. Sie können auch noch andere therapeutisch wertvolle Stoffe enthalten. Auch die Desinfektionsmittel können mit geeigneten Trägerstoffen, wie bekannt, vermischt werden.

Die Dosierung der Wirkstoffe, z.B. der oben besonders hervorgehobenen Aminopapulacandin-Derivate, erfolgt im Prinzip analog derjenigen von anerkannten Antibiotika vom Papulacandin-Typ; sie hängt jedoch auch einerseits von Spezies, Körpergewicht, Alter und individuellem Zustand des Warmblüters, andererseits von der Applikationsweise und insbesondere von der jeweiligen Empfindlichkeit des

Krankheitserregers ab, die man im Routine-Test in bekannter Weise für jeden individuellen Erkrankungsfall feststellen kann.

Die Erfindung betrifft auch eine Methode zur Tötung oder Wachstumsbehinderung (d.h. Inhibition) eines gegen mindestens ein der erfindungsgemässen Aminopapulacandin-Derivate empfindlichen Mikroorganismus, welche durch die Behandlung dieses Mikroorganismus oder eines durch diesen Mikroorganismus infizierten Medium mit einer antimikrobiell wirksamen Dosis eines der erfindungsgemässen Aminopapulacandin-Derivate charakterisiert ist. - Unter der Bezeichnung "eine antimikrobiell wirksame Dosis" ist eine solche Menge des Wirkstoffes zu verstehen, die zu einer wirkungsvollen Inhibition des betreffenden zu behandelnden Mikroorganismus ausreicht.

Die nachfolgenden Beispiele illustrieren die obenbeschriebene Erfindung; sie sollen jedoch diese in ihrem Umfang in keiner Weise einschränken. Temperaturen werden in Celsiusgraden angegeben. Die Zusammensetzung von Lösungsmittelgemischen ist im Volumenverhältnis angegeben.

Beispiel 1: 12-O-(4-Nitrobenzyl)-11-phenylselenoimino-Papulacandin B

Eine Lösung von 10 g 12-O-(4-Nitrobenzyl)-Papulacandin B in 250 ml Tetrahydrofuran wird bei +5° unter Rühren mit 13,7 g Hexamethyldisilazan und nach 5 Minuten mit 2,7 g Phenylseleminsäureanhydrid versetzt, wobei sich die Lösung sofort tiefrot färbt. Nach 5 Minuten wird die Kühlung abgestellt und das Gemisch noch weitere 15 Minuten bei Raumtemperatur weiter gerührt, bis im Dünnschichtchromatogram kein Ausgangsmaterial nachweisbar ist. Die Reaktionslösung wird auf Eiswasser gegossen und dreimal mit Ethylacetat extrahiert. Die vereinigten organischen Extrakte werden zweimal mit Wasser gewaschen, über Natriumsulfat getrocknet und zur Trockne eingedampft. Der Eindampfrückstand wird auf eine Säule von 500 g Silicagel aufgetragen und mit Gemischen von Chloroform mit steigenden Mengen von 2-10 % Methanol eluiert. Durch Eindampfen von entsprechenden Fraktionen wird 12-O-(4-Nitrobenzyl)-11-phenylselenoimino-Papulacandin B als tiefrotes Pulver in zwei Portionen (3,4 g und 1,9 g) erhalten.

Beispiel 2: 11-Amino-12-O-(4-nitrobenzyl)-Papulacandin B

Eine Lösung von 2,6 g 12-O-(4-Nitrobenzyl)-11-phenylselenoimino-Papulacandin B in 50 ml Chloroform wird mit 3 ml Triethylamin versetzt und bei Raumtemperatur während 5 Minuten mit einem Strom von Schwefelwasserstoff bis zur fast vollständigen Entfärbung behandelt. Die Reaktionslösung wird im Vakuum schonend zur Trockne eingedampft, der Rückstand auf eine Säule von 250 g Silicagel aufgetragen und mit Gemischen von Chloroform mit steigenden Mengen (5-20 %) Methanol eluiert. Durch Eindampfen entsprechender Fraktionen und Umfällen aus Aceton-Ether erhält man 2,1 g 11-Amino-12-O-(4-Nitrobenzyl-Papulacandin B als schwach gelb gefärbtes, amorphes Pulver.

UV-Spektrum: (Ethanol): $\lambda$max ($\varepsilon$): 205 (63800), 239 (48000), 264 (57100)

IR-Spektrum: (KBr): 3500, 2950, 2900, 1710, 1615, 1525, 1460, 1345, 1310, 1265, 1175, 1150, 1065, 1040, 1005 $cm^{-1}$.

Beispiel 3: 12-O-Methyl-11-phenylselenoimino-Papulacandin B

Eine Lösung von 5 g Papulacandin B-12-Methylether in 80 ml Tetrahydrofuran wird bei Raumtemperatur unter Rühren mit 4 g Hexamethyldisilazan und nach 5 Minuten mit 4,8 g Phenylseleninsäureanhydrid versetzt, wobei sich die Lösung sofort tiefrot färbt. Die Reaktionslösung wird weitere 7 Minuten bei Raumtemperatur gerührt, auf Eiswasser gegossen und dreimal mit Ethylacetat extrahiert. Die vereinigten organischen Phasen werden zweimal mit Wasser gewaschen, über Natriumsulfat getrocknet und zur Trockne eingedampft. Der Rückstand wird auf eine Säule mit 200 g Siligacel aufgetragen und mit Gemischen von Chloroform mit steigenden Mengen (2-10 %) Methanol eluiert. Durch Eindampfen entsprechender Fraktionen wird 2,0 g 12-O-Methyl-11-phenylselenoimino-Papulacandin B als amorphes tiefrotes Pulver erhalten.

Beispiel 4: 11-Amino-12-O-methyl-Papulacandin B

Eine Lösung von 2 g 12-O-Methyl-11-phenylselenoimino-Papulacandin B in 100 ml Chloroform wird mit 2,3 ml Triethylamin versetzt. Dann wird unter Rühren bei Raumtemperatur während 10 Minuten ein Strom von Schwefelwasserstoff eingeleitet, bis sich die vormals tiefrote Lösung weitgehend entfärbt. Die Reaktionslösung wird im Vakuum schonend zur Trockne eingedampft und der Eindampfrückstand auf einer Säule mit 120 g Silicagel chromatographiert. Durch Eluieren mit Chloroform mit steigenden Mengen (5-20 %) Methanol und Eindampfen der entsprechenden Fraktionen wird ein Rohprodukt erhalten, welches nach Fällen aus Aceton/Ether 1 g 11-Amino-12-O-methyl-Papulacandin B als schwach gelb gefärbtes, amorphes Pulver ergibt.

UV-Spekturm (Ethanol): $\lambda$ max. ($\varepsilon$): 238 (41 200), 265 (37 200), 294 (Schulter)

IR-Spektrum (KBr): 3500, 2950, 1705, 1620, 1525, 1460, 1385, 1345, 1310, 1265, 1150, 1065 $cm^{-1}$.

Beispiel 5: 11-Acetylamino-12-O-(4-nitrobenzyl)-Papulacandin B

Eine Lösung von 150 mg 11-Amino-12-O-(4-nitrobenzyl)-Papulacandin B in 2 ml Methanol wird mit 2 ml Essigsäureanhydrid versetzt und 2 Stunden bei 0°C stehen gelassen. Die Lösung wird im Hochvakuum schonend zur Trockne eingedampft und der Eindampfrückstand auf einer präparativen Silicagel-Dickschichtplatte mit einem Gemisch von Chloroform-Methanol (4:1) als Laufmittel chromatographiert. Nach Isolierung des Produkts aus der entsprechenden Zone und Umfällen aus Aceton-Ether wird 100 mg 11-Acetylamino-12-O-(4-nitrobenzyl)-Papulacandin B als schwach gelb gefärbtes amorphes Pulver erhalten. Das $^{13}C$-NMR-Spektrum entspricht der angegebenen Struktur, u.a. durch die folgenden Maxima (in p.p.m.) C(8): 140,8; C(9): 118,2; C(10): 155,2; C(11): 112,9; C(12): 153,9; C(13): 107,9.

Beispiel 6: 11-Acetylamino-Papulacandin B

Eine Lösung von 110 mg 11-Acetylamino-12-O-(4-nitrobenzyl)-Papulacandin B in 4 ml Methanol und 14 ml Essigsäure wird mit 220 mg Zink-Pulver versetzt und während 20 Minuten bei 0°C intensiv gerührt. Die Reaktionslösung wird über Kieselguhr filtriert,das Filtrat zur Trockne eingedampft und der Eindampfrückstand auf einer präparativen Silicagel-Dickschichtplatte mit einem Gemisch von Chloroform-Methanol (4:1) als Laufmittel chromatographiert, wodurch 15 mg amorphes 11-Acetylamino-Papulacandin B erhalten wird.

Beispiel 7: 11-Mesylamino-12-O-(4-nitrobenzyl)-Papulacandin B

Eine Lösung von 300 mg 11-Amino-12-O-(4-nitrobenzyl)-Papulacandin B in 10 ml Tetrahydrofuran mit 20 Tropfen Pyridin wird mit 47 mg Mesylchlorid versetzt und 15 Stunden bei Raumtemperatur stehen gelassen. Die Reaktionslösung wird auf Eiswasser gegossen, mit Natriumchlorid gesättigt und dreimal mit Ethylacetat extrahiert. Die vereinigten organischen Extrakte werden mit Wasser gewaschen, über Natriumsulfat getrocknet und eingedampft. Der Rückstand (340 mg) wird auf präparativen Silicagel-Dickschichtplatten mit einem Gemisch von Chloroform-Methanol (4:1) als Laufmittel chromatographiert. Es resultiert 205 mg 11-Mesylamino-12-O-(4-nitrobenzyl)-Papulacandin B als farbloses, amorphes Pulver, dessen $^{13}C$-NMR-Spektrum im Einklang mit der angegebenen Struktur u.a. die folgenden Maxima (in p.p.m.) aufweist: C(8): 143,0; C(9): 118,9; C(10): 157,0; C(11): 111,8; C(12): 154,8; C(13): 101,2; aromat. Signale: 149,9; 146,0; 128,9; 124,7.

Beispiel 8: 11-Mesylamino-Papulacandin B

Eine Lösung von 200 mg 11-Mesylamino-12-O-(4-nitrobenzyl)-Papulacandin B in 3 ml Methanol und 7 ml Eisessig wird mit 400 mg Zink-Pulver versetzt, 40 Minuten bei Raumtemperatur intensiv geschüttelt und über Kieselguhr filtriert. Das Filtrat wird schonend zur Trockne eingedampft und der Rückstand auf einer Säule mit 15 g Silicagel chromatographiert. Durch Eluieren mit Gemischen von Chloroform mit steigenden Mengen (5-20 %) Methanol erhält man Fraktionen, die nach der üblichen Verarbeitung 80 mg 11-Mesylamino-Papulacandin B als schwach gelb gefärbtes, amorphes Pulver ergeben. Im Einklang mit der angegebenen Struktur zeigt das Produkt u.a. die folgenden Maxima(in p.p.m.) im $^{13}C$-NMR-Spektrum: C(8): 142,4; C(9): 117,2; C(10): 156,8; (C11) 110,6; C(12) 153,7; C(13) 103,8.

Beispiel 9: 12-O-(4-Nitrobenzyl)-11-N'-(4-nitrobenzyloxycarbonyl)-glycylamino-Papulacandin B

Eine Lösung von 1 g 11-Amino-12-O-(4-nitrobenzyl)-Papulacandin B in 80 ml Tetrahydrofuran wird der Reihe nach mit 680 mg Dicyclohexylcarbodiimid,880 mg N-(4-Nitrobenzyloxycarbonyl)-glycin und 400 mg N-Ethylmorpholin versetzt und 3,5 Stunden bei Raumtemperatur gerührt. Der ausgefallene feine Niederschlag wird abfiltriert, das Filtrat mit Wasser versetzt und die wässrige Lösung fünfmal mit Ethylacetat extrahiert. Die vereinigten organischen Phasen werden mit Wasser gewaschen, über Natriumsulfat getrocknet und zur Trockne eingedampft. Der Rückstand wird an einer Säule mit 20 g Silicagel chromatographiert. Durch Eluieren mit Gemischen von Chloroform mit steigenden Mengen

(10-100 %) Methanol wird aus Fraktionen mit Chloroform-Methanol (1:1) und mit Methanol allein 850 mg angereichertes Produkt erhalten, welches nach nochmaliger Reinigung an präparativen Silicagel-Dickschichtplatten (4 Platten, 20x100 cm, 1,5 mm Schichtdicke) mit einem Gemisch von Chloroform-Methanol-Essigsäure-Wasser (80:18:1:1) als Laufmittel 425 mg 12-O-(4-Nitrobenzyl)-11-N'-(4-nitrobenzyloxycarbonyl)-glycylamino-Papulacandin B als amorphes, farbloses Pulver ergibt.
Im Einklang mit der angegebenen Struktur zeigt das Produkt u.a. die folgenden Maxima im $^{13}C$-NMR-Spektrum (in p.p.m.): (NH-$COCH_2$-) 171,1; NH-$COOCH_2$) 158,7; C(8) ~ 140; C(9) 118,2; C(10) 154,0; C(11) 112,3; C(12) 155,6; C(13) 101,8; aromatische Signale: 66,4; 124,6; 128,9; 129,1; 146,2; 148,8.

Beispiel 10: 11-Glycylamino-Papulacandin B

Eine Lösung von 320 g 12-O-(4-Nitrobenzyl)-11-N'-(4-nitrobenzyloxycarbonyl)-glycylamino-Papulacandin B in 9 ml Methanol und 21 ml Eisessig wird mit 640 mg Zink-Pulver versetzt, 30 Minuten bei Raumtemperatur geschüttelt und über Kieselguhr filtriert. Das Filtrat wird im Hochvakuum schonend zur Trockne eingedampft und der Rückstand an einer Säule von 120 g Sephadex® LH-20 chromatographiert. Die mit Methanol eluierten Fraktionen ergeben 285 mg angereichertes Produkt, das nach nochmaliger Chromatographie an Sephadex® LH-20 95 mg 11-Glycylamino-Papulacandin B als farbloses, amorphes Pulver liefert.

Beispiel 11: 12-O-(4-Nitrobenzyl)-11-(2-oxoimidazolidincarboxamido)-Papulacandin B

Eine Lösung von 1,2 g 11-Amino-12-O-(4-nitrobenzyl)-Papulacandin B in 40 ml Tetrahydrofuran wird mit 204 mg 2-Oxoimidazolidinyl-1-carbonsäurechlorid und 1 ml Pyridin versetzt und 1,5 Stunden bei 0°C gerührt. Die Reaktionslösung wird mit Eiswasser verdünnt, mit Natriumchlorid gesättigt und dreimal mit Ethylacetat extrahiert. Die vereinigten organischen Extrakte werden mit Wasser gewaschen, über Natriumsulfat getrocknet und zur Trockne eingedampft. Der Rückstand

wird an einer Säule mit 100 g Silicagel chromatographiert. Durch Eluieren mit Chloroform mit steigenden Mengen (10-20 %) Methanol werden Fraktionen erhalten, die nach Eindampfen 875 mg 12-O-(4-Nitrobenzyl)-11-(2-oxoimidazolidincarboxamido)-Papulacandin B als schwach gelb gefärbtes, amorphes Pulver ergeben. Im Einklang mit der angegebenen Struktur weist das $^{13}C$-NMR-Spektrum u.a. die folgenden Maxima (in p.p.m.) auf: C(8): 140,0; C(9): 118,4; C(10): 154,3; C(11): 112,9; C(12): 153,7; C(13): 101,9; aromatische Signale: 148,7; 146,2; 128,9; 124,6.

<u>Beispiel 12:</u> <u>11-(2-Oxoimidazolidincarboxamido)-Papulacandin B</u>

Eine Lösung von 875 mg 12-O-(4-Nitrobenzyl)-11-(2-oxoimidazolidin)-carboxamido-Papulacandin B in 12 ml Methanol und 30 ml Eisessig wird mit 1760 mg Zink-Pulver versetzt, 45 Minuten bei Raumtemperatur intensiv geschüttelt und über Kieselguhr filtriert. Das Filtrat wird im Hochvakuum schonend zur Trockne eingedampft und der Rückstand an einer Säule mit 100 g Silicagel chromatographiert. Durch Eluieren mit Gemischen von Chloroform mit steigenden Mengen (10-20 %) Methanol werden Fraktionen erhalten, aus welchen durch Eindampfen 450 mg 11-(2-Oxoimidazolidincarboxamido)-Papulacandin B als farbloses, amorphes Pulver resultiert. - Im Einklang mit der angegebenen Struktur weist das $^{13}C$-NMR-Spektrum u.a. die folgenden Maxima (in p.p.m.) auf: C(8): 140,3; C(9): 116,7; C(10): 154,7; C(11): 111,6; C(12): 153,9; C(13): 104,3; -NH-CO-N< 152,1; 160,2.

<u>Beispiel 13:</u> <u>12-O-(4-Nitrobenzyl)-11-[L-2-(4-nitrobenzyloxycarbonyl)-1-(4-nitrobenzyloxycarbonylamino)-ethoxycarbonylamino]-Papulacandin B</u>

Eine Lösung von 100 mg 12-O-(4-Nitrobenzyl)-Papulacandin B in 5 ml Tetrahydrofuran mit 10 Tropfen Pyridin wird mit 45 mg O-Chloroformyl-N-(4-nitrobenzyloxycarbonyl)-L-serin-4-nitrobenzylester versetzt und 45 Minuten bei 0°C gerührt. Die Reaktionslösung wird auf Eiswasser gegossen, mit Natriumchlorid gesättigt und dreimal mit Ethylacetat extrahiert. Die vereinigten organischen Extrakte werden mit Wasser gewaschen, über Natriumsulfat getrocknet und im Vakuum eingedampft. Der Rückstand wird an einer präparativen Silicagel-Dickschichtplatte mit einem Gemisch von Chloroform-Methanol (4:1) als Laufmittel chromatographiert. Es resultieren 137 mg der Titel-Verbindung (Formel I, Ac = $-CO.O.CH_2\underset{NHCO.O.Nbz}{CH}CO.O.Nbz$, R = Nbz; Nbz = 4-Nitrobenzyl).

Beispiel 14: 12-O-Methyl-11-[L-2-(4-nitrobenzyloxycarbonyl)-1-(4-nitrobenzyloxycarbonylamino)-ethoxycarbonylamino]-Papulacandin B

Eine Lösung von 770 mg 11-Amino-12-O-methyl-Papulacandin B in 25 ml Tetrahydrofuran wird mit 360 mg O-Chloroformyl-N-(4-nitrobenzyloxycarbonyl)-L-serin-4-nitrobenzylester und 0,3 ml Pyridin versetzt und 45 Minuten bei +5°C gerührt. Die Reaktionslösung wird mit Eiswasser versetzt, mit Natriumchlorid gesättigt und fünfmal mit Ethylacetat extrahiert. Die vereinigten organischen Extrakte werden mit Wasser gewaschen, über Natriumsulfat getrocknet und zur Trockne eingedampft. Der Rückstand wird an einer Säule mit 20 g Silicagel chromatographiert. Durch Eluieren mit Gemischen von Chloroform mit steigendem Anteil (5-20 %) Methanol werden Fraktionen erhalten, die nach Eindampfen 700 mg der Titel-Verbindung als schwach gelb gefärbtes amorphes Pulver ergeben (Formel I, Ac = $-CO.O.CH_2\underset{NHCO.O.Nbz}{CH}CO.O.Nbz$, R = $CH_3$; Nbz = 4-Nitrobenzyl).

Beispiel 15: 12-O-Methyl-11-(3-O-serinyl)-carbonylamino-Papulacandin B

Eine Lösung von 700 mg 12-O-Methyl-11-[L-2-(4-nitrobenzyloxycarbonyl)-1-(4-nitrobenzyloxycarbonylamino)-ethoxy-carbonylamino]-Papulacandin B (hergestellt nach Beispiel 14) in 9 ml Methanol und 21 ml Essigsäure wird mit 1,4 g Zink-Pulver versetzt, 45 Minuten bei Raumtemperatur geschüttelt und über Kieselguhr filtriert. Das Filtrat wird im Hochvakuum schonend zur Trockne eingedampft und der Rückstand an einer Säule mit 70 g Silicagel UPC-12® (reversed phase) mit wässrigem Acetonitril als Eluens chromatographiert. Es resultieren 525 mg angereichertes Material, das auf 5 präparativen Silicagel-Dickschichtplatten weiter aufgetrennt wird, wobei als Laufmittel ein Gemisch von

n-Butanol-Essigsäure-Wasser (11:3:7) dient. Die Hauptzone (580 mg) wird noch an einer Säule von 100 g Sephadex® LH-20 chromatographiert, wodurch 80 mg der Titel-Verbindung [Formel I, Ac = $-CO.O.CH_2CH(NH_2)COOH$(-L-); R = $CH_3$] als farbloses, amorphes Pulver resultieren.

Beispiel 16: 11-Acetylamino-12-O-methyl-Papulacandin B

Ein Gemisch von 450 mg 11-Amino-12-O-methyl-Papulacandin B, 2 ml Methanol und 1 ml Essigsäureanhydrid wird 30 Minuten bei 0°C gerührt und schonend zur Trockne eingedampft. Der Rückstand wird an einer präparativen Silicagel-Dickschichtplatte mit Chloroform-Methanol (4:1) chromatographiert. Es resultieren 70 mg 11-Acetylamino-12-O-methyl-Papulacandin B, dessen $^{13}C$-NMR-Spektrum u.a. die folgenden Maxima (in p.p.m.) aufweist: C(8): 141,0; C(9): 117,7; C(10): 155,7; C(11): 112,4; C(12): 146,7; C(13): 100,5; $-COCH_3$: 172,5; $-COCH_3$: 22,6.

Beispiel 17: 12-O-Methyl-11-[N'-(4-nitrobenzyloxycarbonyl)-glycylamino]-Papulacandin B

Eine Lösung von 52 mg 11-Amino-12-O-methyl-Papulacandin B in 4 ml Tetrahydrofuran wird der Reihe nach mit 34 mg Dicyclohexylcarbodiimid, 44 mg N-(4-Nitrobenzyloxycarbonyl)-glycin und 20 ml N-Ethylmorpholin versetzt. Die Reaktionslösung wird 3 Stunden bei Raumtemperatur gerührt und filtriert. Das Filtrat wird mit Wasser versetzt und die wässrige Phase fünfmal mit Ethylacetat extrahiert. Die zur Trockne eingedampften organischen Extrakte werden an einer praparativen Silicagel-Dickschichtplatte chromatographiert, wobei als Laufmittel Chloroform-Methanol (4:1) verwendet wird. Es resultiert 40 mg 12-O-Methyl-11-[N'-(4-nitrobenzyloxycarbonyl)-glycylamino]-Papulacandin B.

Beispiel 18: 11-Glycylamino-12-O-methyl-Papulacandin B

Eine Lösung von 40 mg 12-O-Methyl-11-[N'-(4-nitrobenzyloxycarbonyl)-glycylamino]-Papulacandin B in 1 ml Methanol und 3 ml Essigsäure wird mit 80 mg Zink-Pulver versetzt, 30 Minuten bei Raumtemperatur geschüttelt und über Kieselguhr filtriert. Das Filtrat wird im Hochvakuum

schonend zur Trockne eingedampft und auf einer Silicagel-Dickschichtplatte mit n-Butanol-Eisessig-Wasser (11:3:7) als Laufmittel chromatographiert. Es resultiert 22 ml 11-Glycylamino-12-O-methyl-Papulacandin B.

Beispiel 19: Pharmazeutisches Präparat in Form eines Gels zur Behandlung von Mykosen enthaltend 11-(2-Oxoimidazolidincarboxamido)-Papulacandin B (weiterhin als Wirkstoff bezeichnet).

Gel enthaltend 0,05 % Wirkstoff

Zur Herstellung von 5 Liter Gel vermischt man 100 g hochviskose Methylcellulose mit 500 g Propylenglykol und 3,25 ml Aqua conservans und lässt das Gemisch zu einem homogenen Schleim ausquellen. Dann wird eine Suspension von 2,5 g Wirkstoff in 1 Liter Aqua conservans zugemischt. Schliesslich wird mit Aqua conservans auf 5 Liter ergänzt, sorgfältig gemischt und das erhaltene Gel in Tuben abgefüllt.

Unter Aqua conservans wird eine wässrige Lösung von 0.07 % p-Hydroxybenzoesäure-methylester (Methylparaben) und 0.03 % p-Hydroxybenzoesäure-propylester (Propylparaben) verstanden.

Beispiel 20: Pharmazeutisches Präparat zur Behandlung von Pilz-Infektionen enthaltend 11-(2-Oxoimidazolidincarboxamido)-Papulacandin B

Gele enthaltend 0,5 - 1 % des Wirkstoffes werden wie in Beispiel 19 unter Verwendung der diesbezüglichen nötigen Menge desselben Papulacandin B Wirkstoffes hergestellt.

0054514



Patentansprüche

1. Verfahren zur Herstellung N-acylierter 11-Aminoderivate des Papulacandins A und B der Formel I

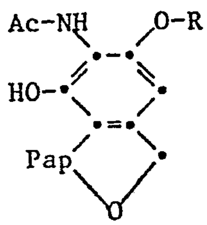

(I),

worin Pap den restlichen Teil des Papulacandins A oder B, R Methyl oder Wasserstoff und Ac den Acylrest einer organischen Säure bedeutet, einschliesslich Salze von Verbindungen mit salzbildenden Eigenschaften, dadurch gekennzeichnet, dass man ein entsprechendes 11-Aminopapulacandin-Derivat, gegebenenfalls unter vorübergehendem Schutz einer in 12-Stellung vorhandenen freien Hydroxylgruppe acyliert und wenn erwünscht, ein erhaltenes Produkt mit salzbildenden Eigenschaften als Salz isoliert.

2. Ein N-acyliertes 11-Aminoderivat des Papulacandins A oder B der Formel I

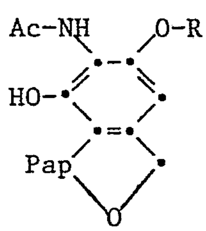

(I),

worin Pap den restlichen Teil des Papulacandins A oder B, R Methyl oder Wasserstoff und Ac den Acylrest einer organischen Säure bedeutet, oder ein Salz davon.

3. Eine Verbindung gemäss Anspruch 2, worin in der Formel I Pap für $Pap_B$, R für Wasserstoff und Ac für den Acylrest einer in der Natur vorkommenden α-Aminosäure steht, oder ein Salz davon.

4. Eine Verbindung gemäss Anspruch 2, worin in der Formel I Pap für $Pap_B$, R für Wasserstoff oder Methyl und Ac für ein Niederalkanoyl steht.

5. Eine Verbindung gemäss Anspruch 2, worin in der Formel I Pap für $Pap_B$, R für Wasserstoff und Ac für den Acylrest einer gegebenenfalls substituierten Niederalkansulfonsäure steht.

6. 11-Mesylamino-Papulacandin B.

7. Eine Verbindung gemäss Anspruch 2, worin in der Formel I Pap für $Pap_B$, R für Wasserstoff oder Methyl und Ac für den Acylrest $Ac_t$ der Formel

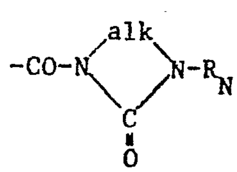

$(Ac_t)$

steht, worin $R_N$ Wasserstoff, Formyl, Niederalkoxycarbonyl, Carbamoyl, Hydroxyniederalkyl oder ein Hydrocarbyl mit höchstens 8 C-Atomen und alk einen Alkylenrest mit 2-8 C-Atomen bedeutet, der die beiden Stickstoffatome voneinander durch mindestens 2 und höchstens 4 C-Atome trennt.

8. 11-(2-Oxoimidazolidin-carboxamido)-Papulacandin B.

9. Verfahren zur Herstellung von 11-Aminopapulacandin-Derivaten der Formel II

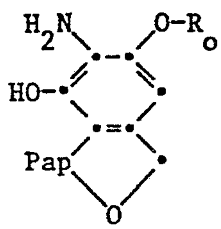

(II)

worin Pap den restlichen Teil des Papulacandins A oder B und $R_o$ Methyl oder eine Hydroxyl-Schutzgruppe Y bedeutet, sowie ihrer Säureadditionssalze, dadurch gekennzeichnet, dass man ein Papulacandin-Derivat der Formel IV

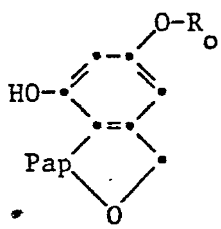

(IV)

worin Pap und $R_o$ die oben genannten Bedeutungen haben,
a) mit einem Arylseleninsäureanhydrid und Hexaniederalkyldisilazan umsetzt und nachher b) mit einem Reduktionsmittel behandelt und, wenn erwünscht, eine erhaltene Base in ein Säureadditionssalz und/oder ein erhaltenes Salz in die freie Base umwandelt.

10. Ein 11-Aminopapulacandin-Derivat der Formel II

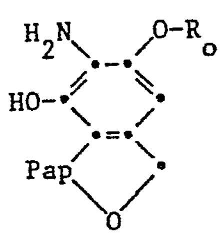

(II)

worin Pap den restlichen Teil des Papulacandins A oder B und $R_o$ Methyl oder eine Hydroxyl-Schutzgruppe Y bedeutet, sowie ein Säureadditionssalz davon.

11. Eine Verbindung gemäss Anspruch 10, worin in der Formel II $R_o$ für Methyl steht.

12. 11-Amino-12-O-methyl-Papulacandin B.

13. Eine Verbindung gemäss einem der Ansprüche 2 bis 8, 11 und 12 zur Verwendung als Antibiotikum.

14. Ein pharmazeutisches Präparat enthaltend eine Verbindung gemäss einem der Ansprüche 2 bis 8 und 11 bis 13 als Wirkstoff.

Europäisches Patentamt

# EUROPÄISCHER RECHERCHENBERICHT

0054514

Nummer der Anmeldung

EP 81 81 0489

| EINSCHLÄGIGE DOKUMENTE | | | KLASSIFIKATION DER ANMELDUNG (Int Cl.3) |
|---|---|---|---|
| Kategorie | Kennzeichnung des Dokuments mit Angabe, soweit erforderlich, der maßgeblichen Teile | betrifft Anspruch | |
| YD | JOURNAL OF ANTIBIOTICS, Band XXXIII, Nr. 9, September 1980, Seiten 967-978 Tokyo, JP. P. TRAXLER et al.: "Papulacandins, a new family of antibiotics with antifungal activity. Structures of papulacandins A,B,C and D" * Seiten 967-968 * | 2,6,8, 10,14 | C 07 H 19/00<br>A 61 K 31/70 |
| Y | DE - A - 2 739 974 (CIBA GEIGY) * Ansprüche * | 2,6,8, 10,14 | RECHERCHIERTE SACHGEBIETE (Int Cl.3) |
| D | & BE - A - 858 492 | | C 07 H 19/00<br>A 61 K 31/00 |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 08-03-1982 | VERHULST |